# EUROPÄISCHE PATENTANMELDUNG

(11) **EP 4 567 821 A1**
(43) Veröffentlichungstag der Anmeldung: **11.06.2025**
(21) Anmeldenummer: 23215401.3
(22) Anmeldetag: 10.12.2023
(51) Int. Cl.: G16H 20/00, G16H 50/50, G16H 50/20

(54) **SYSTEM ZUM ERSTELLEN EINES DIGITALEN ZWILLINGS EINES INDIVIDUUMS, EIN COMPUTERIMPLEMENTIERTES VERFAHREN DAFÜR UND EIN COMPUTERPROGRAMMPRODUKT SOWIE EIN COMPUTERLESBARES SPEICHERMEDIUM DAFÜR**

(71) Anmelder: ACMIT Gmbh, 2700 Wiener Neustadt (AT)
(72) Erfinder: Dellantoni, Nikolaus, 2504 Sooss (AT); Bernhard, Nussbaumer, 2721 Bad Fischau (AT)
(74) Vertreter: Neuhold, Alfred

(57) **Zusammenfassung**

Die Erfindung betrifft ein System 15 zum Erstellen eines digitalen Zwillings 22 eines Individuums 16 mithilfe von Daten umfassend ein Datenauswertemodul 17, welches zum Datenaustausch mit zumindest einem Datenspeichermedium 19 verbunden ist, wobei das Datenauswertemodul dazu ausgebildet ist, beim Erstellen des digitalen Zwillings 22 die folgenden Schritte auszuführen: Bereitstellen eines Templates 20 für einen digitalen Zwilling eines definierten Individuums; Festlegen eines Datenvervollständigungskriteriums 21 für den digitalen Zwilling des definierten Individuums; Aufnehmen von Daten des definierten Individuums in den digitalen Zwilling; Analysieren der aufgenommenen Daten des definierten Individuums im digitalen Zwilling; Vergleichen der analysierten Daten mit dem festgelegten Datenvervollständigungskriterium und Festlegen von fehlenden Dateneinträgen für den digitalen Zwilling auf Basis des Datenvervollständigungskriteriums; Auswählen von Daten aus statistischen medizinischen Daten von mehreren Individuen 23 aus dem Datenspeichermedium; Ergänzen der fehlenden Dateneinträge im digitalen Zwilling des definierten Individuums mit den ausgewählten Daten. Weiters umfasst die Erfindung ein computerimplementiertes Verfahren.

## Beschreibung

Die vorliegende Erfindung betrifft ein System zum Erstellen eines digitalen Zwillings eines Individuums gemäss dem Patentanspruch 1, ein computerimplementiertes Verfahren gemäss dem Patentanspruch 13, ein Computerprogrammprodukt gemäss dem Patentanspruch 14 sowie ein computerlesbares Speichermedium gemäss dem Patentanspruch 15.

### Technologischer Hintergrund

Es wird bereits heutzutage versucht, die in der Industrie schon übliche Idee des digitalen Zwillings auf die Anwendung beim Menschen zu übertragen. Die Schwierigkeit dabei ist die Tatsache, dass im Gegensatz zu technischen Systemen jeder Mensch individuell anders ist, sowohl physisch als auch in der Reaktion auf Therapien und Medikamente. Darüber hinaus ist die bekannte Datenlage bei einem einzelnen Menschen zu dünn, um einen relevanten Mehrwert zu erzeugen, da - vorwiegend aus Kostengründen und ethischen Gründen - keine systematische medizinische Datenerhebung bei lebenden Menschen stattfindet (d.h. keine Versuche oder risikobehaftete Untersuchungen an Menschen ohne medizinische Notwendigkeit).

Bei heutigen klinischen Studien an lebenden Menschen stellen die Ergebnisse nur eine Näherung dar, da die Studiengruppe immer nur stellvertretend für einen einzelnen Menschen mit bestimmten Eigenschaften ist. Die gesamten klinischen Nachweise in der Medizin basieren auf statistischer Häufigkeit, da jeder Mensch anders ist. In zunehmendem Maße werden klinische Studien und Tierversuche durch sogenannte in-silico-trials ersetzt (siehe Beschluss FDA aus dem Jahre 2022).

Aus dem Stand der Technik ist die WO 2022/040536 A1 bekannt. Es wird ein Verfahren zur Bearbeitung eines medizinischen Anspruchs, ein System und ein Computerprogrammprodukt dafür offenbart, wobei das Verfahren folgende Schritte umfasst: Den Empfang eines medizinischen Anspruchs, der einem Patienten zugeordnet ist zur Erstellung eines digitalen Zwillings des Patienten; die mathematische Analyse, ob der medizinische Anspruch mit dem digitalen Zwilling übereinstimmt; und die Ausgabe eines Hinweises auf eine mögliche Nichterfüllung, wenn der medizinische Anspruch nicht mit dem digitalen Zwilling übereinstimmt. Die mathematische Analyse prüft, ob der medizinische Anspruch mit dem digitalen Zwilling übereinstimmt, und beinhaltet die Bestimmung eines Grades, in dem die medizinische Leistung mit dem mathematischen Modell übereinstimmt, und die Ausgabe einer Punktzahl, die diesen Grad angibt. Die mathematische Analyse, ob der medizinische Anspruch mit dem digitalen Zwilling übereinstimmt, kann die Bestimmung beinhalten, ob eine medizinische Leistung, die mit dem medizinischen Anspruch verbunden ist, auf der Grundlage des digitalen Zwillings medizinisch indiziert war und/oder ob eine medizinische Leistung, die mit dem medizinischen Anspruch verbunden ist, mit medizinischen Normen in Bezug auf einen Gesundheitszustand des Patienten übereinstimmt.

Nachteilig an dieser bekannten Lösung ist, dass lediglich der Wahrheitsgehalt eines medizinischen Anspruchs verifiziert wird. Dies wird anhand eines mathematischen Vergleichs mit einem digitalen Zwilling und einem realen Patienten gemacht.

Aus dem Stand der Technik ist die EP 3 646 331 A1 bekannt. Es wird ein Gerät mit einem Prozessor und einem Speicher offenbart. Der Beispielprozessor soll den Speicher entsprechend einem digitalen Zwilling eines ersten Patienten konfigurieren. Der digitale Zwilling des Beispielpatienten soll eine Datenstruktur enthalten, die aus einer Kombination von medizinischen Patientendaten, Bilddaten, genetischen Informationen und historischen Informationen erstellt wurde, wobei die Kombination aus einem oder mehreren Informationssystemen extrahiert, und in der Datenstruktur angeordnet wurde, um eine digitale Darstellung des ersten Patienten zu bilden. Der digitale Zwilling des Beispielpatienten soll für die Abfrage und Simulation durch den Prozessor eingerichtet werden. Der digitale Zwilling des Beispielpatienten soll mit einer oder mehreren Regeln kombiniert werden können, um mit Hilfe des Prozessors eine Empfehlung für ein Gesundheitsergebnis des Patienten auf der Grundlage der Modellierung des digitalen Zwillings des Patienten gemäß den Anweisungen der einen oder mehreren Regeln zu erzeugen.

Aus dem Stand der Technik ist die WO 2019/005185 A1 bekannt. Es ist ein Gerät mit einem digitalen Zwilling eines Patienten und mit einer Datenstruktur offenbart, die aus einer Kombination von elektronischen medizinischen Patientendaten und historischen Informationen erstellt wurde, wobei die Kombination aus Informationssystemen extrahiert, und in der Datenstruktur angeordnet wurde, um eine digitale Darstellung des Patienten zu bilden. Der beispielhafte digitale Zwilling des Patienten ist für Abfragen und Simulationen eingerichtet. Der digitale Zwilling des Beispielpatienten dient dazu, nach einem am Patienten durchgeführten Verfahren Rückmeldungen über den Patienten zu erhalten. Der digitale Zwilling des Beispielpatienten soll das Feedback in den digitalen Zwilling des Patienten einbauen, wenn die Elemente des Verfahrens abgeschlossen sind. Der digitale Zwilling des Beispielpatienten soll das aufgenommene Feedback verarbeiten, um eine Empfehlung für die Weiterbehandlung des Patienten auf der Grundlage der digitalen Darstellung des Patienten einschließlich des aufgenommenen Feedbacks in Bezug auf das durchgeführte Verfahren zu erzeugen und auszugeben.

Nachteilig an den beiden vorgenannten bekannten Lösungen ist, dass fehlende Daten im digitalen Zwilling nicht ergänzt werden und somit ein unvollständiger digitaler Zwilling entsteht.

Aus dem Stand der Technik ist die US 2022/013199 A1 bekannt. Es wird ein computerimplementiertes Verfahren und ein System zur Verwaltung digitaler Zwillinge offenbart, wobei das Verfahren Folgendes umfasst: Sammeln von Daten, die einem oder mehreren Körpergeweben entsprechen; Erzeugen einer oder mehrerer digitaler Zwillingsversionen des einen oder der mehreren Körpergewebe; Identifizieren einer am besten geeigneten digitalen Zwillingsversion der einen oder der mehreren digitalen Zwillingsversionen auf der Grundlage einer Gesundheit und einer Kompatibilität der einen oder der mehreren digitalen Zwillingsversionen; und Verteilen der am besten geeigneten digitalen Zwillingsversion.

Nachteilig an dieser bekannten Lösung ist, dass nur Modelle von digitalen Zwillingen erstellt werden. Eines der erstellten Modelle kann dann für eine medizinische Fragestellung herangezogen werden.

Aus dem Stand der Technik ist die EP 3 809 420 A1 bekannt. Es wird ein System, ein Verfahren und ein Computerprogrammprodukt offenbart. Das System umfasst eine erste Verarbeitungsanordnung, die in mindestens einem ersten Modus eingerichtet ist, und folgende Schritte ausführt: Abrufen eines digitalen Modells von mindestens einem Teil einer Anatomie eines Patienten, Simulieren einer Vielzahl von physikalischen Zuständen des mindestens einen Teils der Anatomie auf der Grundlage der Einstellung eines Satzes von einem oder mehreren Eingangsparametern des Modells und Entwickeln des digitalen Modells auf der Grundlage jedes Satzes von eingestellten Parametern; für jeden simulierten physikalischen Zustand einen oder mehrere physiologische oder anatomische Parameter abzuleiten, die mit dem simulierten mindestens einen Teil der Anatomie assoziiert sind, und die abgeleiteten Parameter in einer Datenspeicheranordnung zusammen mit dem Satz von angepassten Eingabeparametern entsprechend dem simulierten physikalischen Zustand zu speichern, um dadurch einen von dem digitalen Modell getrennten Referenzdatensatz aufzubauen. Es ist eine weitere Verarbeitungsanordnung vorhanden, die mit der ersten Verarbeitungsanordnung identisch und die in mindestens einem zweiten Modus angeordnet ist, um: Empfangen eines Satzes von einem oder mehreren gemessenen Parametern, die sich auf den mindestens einen Teil der Anatomie des Patienten beziehen; Zuordnen der gemessenen Patientenparameter zu einem der Sätze von Modelleingangsparametern, die in dem Referenzdatensatz gespeichert sind, und Abrufen der entsprechenden simulierten physiologischen oder anatomischen Parameter aus dem Referenzdatensatz und Erzeugen der Ausgabe auf der Grundlage des einen oder der mehreren abgerufenen physiologischen oder anatomischen Parameter.

Nachteilig an dieser bekannten Lösung ist, dass hierbei eine reine Optimierung des Speicherplatzes der Daten in der Verarbeitung für einen digitalen Zwilling behandelt wird.

Aus dem Stand der Technik ist die US 2019/198169 A1 bekannt. Es wird eine Vorrichtung zur Auswahl einer medizinischen Behandlung bereitgestellt, die eine Benutzerschnittstelle zum Empfang von Gesundheitsinformationen für einen Patienten umfasst, wobei die Gesundheitsinformationen Symptome eines Zustands enthalten. Das Beispielsystem enthält zusätzlich einen Datenanalysator, um auf historische Patienteninformationen zuzugreifen, die in einer Datenbank für frühere Patienten gespeichert sind, und um einen Zustand auf der Grundlage des Vergleichs historischer Patienteninformationen, die in einer Datenbank für frühere Patienten gespeichert sind, mit den Gesundheitsinformationen für den Patienten zu bestimmen. Die Beispielvorrichtung umfasst auch eine Maschine zum maschinellen Lernen, um mindestens einen Behandlungsplan zu empfehlen, der von der Benutzerschnittstelle präsentiert werden soll, einschließlich: eines Datenanalysealgorithmus-Servers, um Erfolgsraten des mindestens einen Behandlungsplans für den Zustand zu bestimmen, wobei die Erfolgsraten auf den Gesundheitsinformationen für den Patienten und historischen Gesundheitsinformationen basieren; und eines Modellgenerators, um ein Patientenmodell zu erzeugen, wobei das Patientenmodell Auswirkungen des mindestens einen Behandlungsplans und Symptome des Zustands für eine Dauer des Behandlungsplans auf der Grundlage der Erfolgsraten vorhersagt. Die Vorrichtung enthält zusätzlich eine Kommunikationsschnittstelle, um die Planung eines Termins mit einem Kliniker zu erleichtern, nachdem der Patient den Behandlungsplan über die Benutzerschnittstelle ausgewählt hat.

Nachteilig an dieser bekannten Lösung ist, dass die Daten der Datenbank für frühere Patienten im Patientenmodell nicht weiterverwendet werden, sondern nur als Basis für einen Behandlungsplan für den individuellen Patienten dienen.

### Darstellung der Erfindung

Eine Aufgabe der Erfindung besteht darin, mindestens einen der Nachteile des Standes der Technik zu vermeiden. Es soll ein System geschaffen werden, welches einen verbesserten digitalen Zwilling eines Individuums schafft, sodass dieser für klinische Studien anstelle von Lebewesen verwendet werden kann und dadurch risikobehaftete klinische Studien an Lebewesen zumindest deutlich reduzierbar sind. Weiters soll ein computerimplementiertes Verfahren, ein Computerprogrammprodukt sowie ein computerlesbares Speichermedium geschaffen werden, welche zur deutlichen Reduzierung von klinischen Studien an Lebewesen mitwirken.

Diese Aufgabe wird durch die Merkmale der unabhängigen Patentansprüche gelöst. Vorteilhafte Weiterbildungen sind in den Figuren und in den abhängigen Patentansprüchen dargelegt.

Ein erfindungsgemässes System zum Erstellen eines digitalen Zwillings eines Individuums mithilfe von Daten umfasst ein Datenauswertemodul mit zumindest einer Recheneinheit, welches zum Datenaustausch mit zumindest einem Datenspeichermedium verbunden ist, wobei das Datenauswertemodul dazu ausgebildet ist, beim Erstellen des digitalen Zwillings mindestens die folgenden Schritte auszuführen,
a) Bereitstellen eines Templates für einen digitalen Zwilling eines definierten Individuums;
b) Festlegen zumindest eines Datenvervollständigungskriteriums für den digitalen Zwilling des definierten Individuums;
c) Aufnehmen von Daten des definierten Individuums in den digitalen Zwilling;
d) Analysieren der aufgenommenen Daten des definierten Individuums im digitalen Zwilling;
e) Vergleichen der analysierten Daten mit dem festgelegten Datenvervollständigungskriterium und Festlegen von fehlenden Dateneinträgen für den digitalen Zwilling auf Basis des Datenvervollständigungskriteriums;
f) Auswählen von Daten aus statistischen medizinischen Daten von mehreren Individuen aus dem zumindest einen Datenspeichermedium;
g) Ergänzen der fehlenden Dateneinträge im digitalen Zwilling des definierten Individuums mit den ausgewählten Daten.

Dieses System ersetzt bzw. reduziert die Anzahl von klinischen Studien an Lebewesen massiv, da mithilfe des Systems ein digitaler Zwilling mit einer für klinische Studien ausreichenden Datenlage geschaffen wird. Diese digitalen Zwillinge können für klinische Studien anstelle von Lebewesen verwendet werden, sodass risikobehaftete klinische Studien an Lebewesen zumindest deutlich reduzierbar sind. Zur Schaffung des digitalen Zwillings beispielsweise eines bestimmten Menschen werden zunächst existierende Daten dieses Menschen (z.B. aus Krankenakte aber auch andere Informationen wie z.B. Tätigkeit, Wohnorte, usw.) verwendet. Um Datenlücken aufzufüllen, werden dann zusätzlich statistische medizinische Daten von mehreren Individuen verwendet. Fehlende medizinische Daten werden durch existente statistisch relevante Daten möglichst ähnlicher Individuen ersetzt. Statistische medizinische Daten sind somit medizinische Daten von mehreren Individuen, welche statistisch gesehen zum definierten Individuum passen. Damit ist eine Erprobung von neuen Therapien am digitalen Zwilling sowie eine parallele Analyse an vielen Instanzen desselben digitalen Zwillings ermöglicht. Die statistischen medizinischen Daten stammen von vielen unterschiedlichen Individuen aus Datenspeichern von unterschiedlichen Krankenhäusern, Ländern oder Kontinenten.

Durch die Nutzung dieses Systems können beispielsweise an digitalen Zwillingen von Menschen viele Studien mit zumindest gleichwertiger Ergebnisqualität zu heutigen klinischen Studien an lebenden Menschen durchgeführt werden. Für die klinischen Studien können anstelle echter Menschen ein oder mehrere digitale Zwillinge verwendet werden. Ein digitaler Zwilling eines Menschen muss nicht zwangsläufig identisch zum Menschen sein, sondern kann auch statistisch relevante Daten anderer Menschen beinhalten.

Insbesondere werden vor dem Auswählen der Daten aus den statistischen, medizinischen Daten von mehreren Individuen im Schritt f) diese Daten im Datenauswertemodul verifiziert. Damit kann sichergestellt werden, dass nur verifizierte Daten im digitalen Zwilling verwendet werden. Manche klinischen Studien verlangen verifizierte Daten, sodass auch diese klinischen Studien mit dem erzeugten digitalen Zwilling durchführbar sind. Als ein Verifikationskriterium kann beispielsweise das Ausschliessen der Daten eines Individuums mit einer bestimmten Krankheit vorgesehen sein.

Unter einem "digitalen Zwilling" wird hier eine digitale Repräsentation eines Individuums verstanden, beinhaltend zumindest ein Identifikationsmerkmal, bspw. Geburtsdatum, Geschlecht usw., und mindestens ein Informationselement, welche mindestens eine physische Eigenschaft des Individuums beschreiben, wie z.B. Tumorgewebe, Blut, Bildgebungsdaten, etc. Ein Individuum ist hier vorliegend ein lebender Körper oder ein verstorbener Körper eines Menschen oder eines Tieres mit all seinen medizinisch wie auch physisch erfassbaren Parametern.

Der Benutzer des Systems definiert ein Datenvervollständigungskriterium. Unter dem zumindest einen Datenvervollständigungskriterium können Einschränkungen der allgemeinen Zieldefinition, z.B. auf bestimmte Personengruppen (Alter, Geschlecht, Anzahl, etc.) verstanden werden. Beispielsweise verlangt das Datenvervollständigungskriterium, dass vergleichbare statistische medizinische Daten von einer Mindestanzahl von Individuen übereinstimmend vorhanden sein müssen, z.B. dass die ausgewählten Daten aus den statistischen medizinischen Daten zumindest 100 Individuen umfassen, welche Diabetes Typ A aufweisen. Die fehlenden Dateneinträge (bspw. bestimmte Blutwerte) im digitalen Zwilling des definierten Individuums werden nur mit den statistischen Daten der zumindest 100 Individuen ergänzt. Grundsätzlich sollen in dem hier vorliegend offenbarten System nicht alle Daten aus dem Datenspeichermedium für das Ergänzen des digitalen Zwillings verwendet werden, sondern nur jene, die dem Datenvervollständigungskriterium entsprechen. Damit wird beim Erstellen jedes digitalen Zwillings im System nur ein Bruchteil der im Datenspeichermedium vorhandenen Daten genutzt, nämlich die relevanten Daten für den digitalen Zwilling des definierten Individuums.

Neben dem Aufnehmen von Daten des definierten Individuums in den digitalen Zwilling, werden auch insbesondere dessen medizinische Daten aufgenommen. Damit sind die vorhandenen medizinischen Daten des definierten Individuums unmittelbar im digitalen Zwilling einsetzbar.

Insbesondere ist die Reihenfolge der zuvor genannten Schritte a) bis f) festgelegt, sodass die Auswahl der Daten aus den statistischen medizinischen Daten der mehreren Individuen aus dem zumindest einen Datenspeichermedium reproduzierbar erfolgen kann.

Vorzugsweise umfasst das Datenvervollständigungskriterium zumindest eine medizinische Fragestellung. Diese soll datenbasiert beantwortet werden. Die zumindest eine medizinische Fragestellung hat somit einen massgeblichen Einfluss auf die Auswahl der statistischen medizinischen Daten der mehreren Individuen aus dem zumindest einen Datenspeichermedium. Beispielsweise kann die medizinische Fragestellung so formuliert sein, dass die Individuen keine Lungenvorerkrankungen, wie CoV19 oder Keuchhusten, aufweisen dürfen. Derartige Daten von Individuen werden vom System ausgeschlossen. Das Ausschliessen der Daten von Individuen mit gewissen Vorerkrankungen beschleunigt die Auswahl der Daten, und der digitale Zwilling wird nur mit relevanten Daten ergänzt.

Bevorzugterweise ist eine Schnittstelle vorhanden, an der die Daten des digitalen Zwillings für einen Benutzer bereitstellbar sind, wobei der Benutzer neben einer Person auch eine Künstliche Intelligenz oder eine Steuereinrichtung sein kann. Die Schnittstelle ermöglicht eine individuelle Weiterverarbeitung der Daten im digitalen Zwilling durch den Benutzer. Insbesondere ist eine Eingabeeinrichtung vorhanden, sodass der Benutzer zusätzlich Daten eingeben kann bzw. alternativ oder ergänzend das zumindest eine Datenvervollständigungskriterium dem Datenauswertemodul übermitteln kann.

Alternativ oder ergänzend sind die Daten des digitalen Zwillings für einen Benutzer an einer Anzeigeeinrichtung darstellbar. Die Anzeigeeinrichtung ist mit dem System verbunden und umfasst ein Graphikmodul zum graphischen Darstellen des digitalen Zwillings. Der Benutzer kann unmittelbar auf die Daten im digitalen Zwilling zugreifen und diese beliebig weiterverarbeiten. Der Benutzer hat auch die Möglichkeit, die Daten im digitalen Zwilling des definierten Individuums zu verifizieren. Der Benutzer kann zudem weitere Handlungen vornehmen oder Korrekturen im zumindest einen Datenvervollständigungskriterium vornehmen, um zu den gewünschten statistischen medizinischen Daten mit der gewünschten Relevanz zu kommen. Insbesondere umfasst die Anzeigeeinrichtung ein Display, bevorzugt einen Touchscreen, sodass die ausgewählten statistischen medizinischen Daten dem Benutzer bedienerfreundlich zur Verfügung stehen. Ein Touchscreen kann zur Dateneingabe und Datenausgabe verwendet werden. Das Display kann mit dem System verbunden sein. Beispielsweise ist die Anzeigeeinrichtung eine tragbare Einrichtung, wie beispielsweise ein Smartphone oder ein Tablet. Dabei können mehrere Anzeigeeinrichtungen vorhanden sein, sodass die Daten des digitalen Zwillings mehreren Benutzern gleichzeitig zur Verfügung stehen.

Insbesondere sind die Daten des digitalen Zwillings für einen Benutzer an einer Anzeigeeinrichtung animiert, darstellbar. Ein animiertes Darstellen der Daten im digitalen Zwilling ermöglicht es dem Benutzer des Systems, kognitiv die Daten im digitalen Zwilling zu erfassen und zu verarbeiten.

Bevorzugt ist eine Steuerungseinrichtung vorhanden, wobei die Steuerungseinrichtung die Schnittstelle veranlasst, die ausgewählten Daten auszugeben. Damit ist die Ausgabe der ausgewählten Daten automatisch von der Steuerungseinrichtung ausführbar, sodass keine zusätzlichen Schritte vom Benutzer zu tätigen sind. Damit wird das Weiterverarbeiten der Daten im digitalen Zwilling für klinische Studien vereinfacht.

Alternativ oder ergänzend veranlasst die Steuerungseinrichtung die Anzeigeeinrichtung mindestens die ausgewählten Daten auszugeben. Die ausgewählten Daten können dem Benutzer direkt angezeigt werden, sodass der Benutzer die ausgewählten Daten für den digitalen Zwilling verifizieren kann. Damit ist sichergestellt, dass die ausgewählten Daten für den digitalen Zwilling des definierten Individuums vor der Weiterverarbeitung von einem Benutzer verifiziert werden.

Vorzugsweise umfassen die statistischen medizinischen Daten zumindest Daten von verstorbenen Individuen. Unter Daten verstorbener Individuen werden zum einen Autopsiedaten verstanden und zum anderen historische Patientendaten bzw. die Patientenakte eines Verstorbenen, bzw. historische Datensätze, die die Krankengeschichte eines Verstorbenen und alle damit in Verbindung stehenden Daten des verstorbenen Individuums widerspiegeln. Darüber hinaus können diese Daten die Todesursache des Individuums umfassen, welche beispielsweise mit jahrelangem Medikamentengebrauchs zusammenhängen können. Diese medizinischen Daten von Verstorbenen als statistische medizinische Daten verwenden zu können, revolutioniert die Verwendung von Daten für klinische Studien. Durch Nutzung der riesigen Datenmenge Verstorbener, können an mindestens einem bzw. mehreren digitalen Zwillingen von Individuen viele klinische Studien mit zumindest gleichwertiger Ergebnisqualität durchgeführt werden, als würden diese Studien an Lebewesen durchgeführt.

Alternativ oder ergänzend umfassen die statistischen medizinischen Daten zumindest Daten von lebenden Individuen. Diese lebenden Individuen weisen medizinische Daten auf, welche statistisch gesehen zum digitalen Zwilling des definierten Individuums passen. Beispielsweise stimmen die medizinischen Daten im Datenspeichermedium aufgrund des Alters, des Geschlechts, der Vorerkrankungen und des Blutbildes mit dem digitalen Zwilling des definierten Individuums überein, sodass eine weitere Verifikation nicht notwendig ist. Unter Datensätzen von lebenden Individuen werden historische Patientendaten bzw. die Patientenakte bzw. historische Datensätze verstanden, die die Krankengeschichte und alle damit in Verbindung stehenden Daten des lebenden Individuums widerspiegeln. Beispielsweise kann ein jahrelanger Medikamentenkonsum mit Folgeerkrankungen zusammenhängen. Durch Nutzung der riesigen Datenmenge im Datenspeichermedium können an mindestens einem bzw. mehreren digitalen Zwillingen von Individuen viele klinische Studien mit zumindest gleichwertiger Ergebnisqualität durchgeführt werden, als würden diese Studien an Lebewesen durchgeführt.

Bevorzugterweise wurden die Daten von verstorbenen Individuen mittels zumindest eines medizinischen Datengewinnungsverfahrens generiert. Alternativ oder ergänzend wurden die Daten von lebenden Individuen mittels zumindest eines medizinischen Datengewinnungsverfahrens generiert. Anerkannte medizinische Datengewinnungsverfahren sind bereits erprobt und benötigen daher keine zusätzliche Verifikation, sodass diese Daten von verstorbenen oder von lebenden Individuen bedenkenlos im digitalen Zwilling des definierten Individuums verwendbar sind. Dies vereinfacht das Auswählen der statistischen medizinischen Daten für den digitalen Zwilling.

Vorzugsweise sind die Daten des definierten Individuums im digitalen Zwilling in einer Datenstruktur im zumindest einen Datenspeichermedium abgelegt. Damit sind die ausgewählten medizinischen Daten einfach auffindbar und verbessert mit dem Datenvervollständigungskriterium verknüpfbar. Beispielsweise sind die medizinischen Daten in Cluster zusammengefasst, sodass diese einfach mit einer Expertendatenbank verknüpfbar sind. Die Datenstruktur ist dem System bekannt, sodass das Ergänzen der fehlenden Dateneinträgen im digitalen Zwilling fehlerlos erfolgen kann.

Bevorzugterweise ist ein Auswertealgorithmus vorhanden. Der Auswertealgorithmus analysiert die aufgenommenen Daten im Schritt d) auf Basis zumindest eines Datenvervollständigungskriteriums. Damit ist sichergestellt, dass die statistischen medizinischen Daten richtig ausgewertet werden, und damit der digitale Zwilling entsprechend der Datenvervollständigungskriterium ergänzt wurde.

Insbesondere ist der Auswertealgorithmus ausgebildet, zumindest einen Biomarker zu identifizieren. Wenn es darum geht, Biomarker für bestimmte Krankheitsbilder zu entwickeln, um diese Krankheiten frühzeitig erkennen zu können, können diese auch durch Analyse der medizinischen Daten Verstorbener (inkludierend auch Daten, als diese Verstorbenen noch gelebt haben) ermittelt werden. Dies erfolgt insbesondere durch die Analyse digitaler Zwillinge dieser Verstorbenen. Daraufhin können weitere Daten (z.B. Biopsie eines Tumors) generiert werden, die zur Validierung genutzt werden können. Hauptvorteil dabei ist, dass bei Verstorbenen Krankheitsbilder erkannt werden können, die beim noch Lebenden nicht sichtbar waren. Eventuell hätten diese Krankheitsbilder durch diverse invasive Untersuchungsmethoden (wie z.B. CT, Biopsien) erkannt werden können, jedoch dürfen aus ethischen Gründen ohne konkreten Verdacht an Lebenden keine solchen invasiven Untersuchungen durchgeführt werden.

Vorzugsweise ist der Auswertealgorithmus ausgebildet, den Vergleich in Schritt e) zu optimieren. Dafür weist der Auswertealgorithmus noch Rahmenbedingungen oder Schranken auf, die den Vergleich verbessern und damit optimieren. Der Auswertealgorithmus kann die zur Verfügung stehenden Daten beispielsweise nach zusätzlichen Parametern, wie etwa Ort und Zeitpunkt eines Ereignisses, insbesondere der Zeitpunkt einer Krankheitsdiagnose beim Individuum, reihen, und damit Daten mit früheren Diagnosezeitpunkten in der Auswahl der statistischen medizinischen Daten bevorzugen. Die Rahmenbedingungen oder Schranken ermöglichen ein weiteres Selektieren der Daten für den digitalen Zwilling.

Bevorzugterweise ist der Auswertealgorithmus ausgebildet, zumindest einen medizinischen Behandlungsvorschlag für das definierte Individuum bereitzustellen. Beliebige Behandlungsoptionen für einen Patienten werden anhand des digitalen Zwillings in Echtzeit validiert, da anhand der Vielzahl an vorhandenen Daten eine mindestens gleich hohe statistische Signifikanz wie bei einer klinischen Studie erreicht werden kann ("in-silico-trial" mit gleich guter Aussage wie ein echter "clinical trial"). Basierend darauf können hohe Kosten und Zeitaufwände für die Durchführung klinischer Studien eingespart werden.

Vorzugsweise ist ein Künstliches Intelligenzmodul (KI) vorhanden, welches auf Basis des Datenvervollständigungskriteriums die Ergänzung des digitalen Zwillings des definierten Individuums mit Daten, insbesondere automatisch, vorschlägt. Die ausgewählten Daten können vom KI-Modul direkt verarbeitet werden, sodass kein Benutzer die ausgewählten Daten für den digitalen Zwilling verifizieren muss. Damit ist sichergestellt, dass die ausgewählten Daten für den digitalen Zwilling des definierten Individuums vor der Weiterverarbeitung auch von einem KI-Modul verifiziert werden können.

Die ausgewählten statistischen medizinischen Daten können wiederum als Trainingsdaten für das KI-Modul dienen, sodass das System intelligent und selbstlernend ist. Damit wird ein System zur Identifikation von statistischen medizinischen Daten geschaffen, welches eine hohe Datenqualität aufweist und die Auswahl der Daten mit hoher Qualität favorisiert. Insbesondere weist das KI-Modul den statistischen medizinischen Daten eine medizinische Relevanz auf Basis des mindestens einen Datenvervollständigungskriteriums zu und verknüpft somit die medizinischen Daten mit dem Datenvervollständigungskriterium. Das KI-Modul kann wiederum ausgebildet sein, den medizinischen Daten eine medizinische Relevanz zuzuordnen, sodass diese bei einer nachfolgenden veränderten Bestimmung eines Datenvervollständigungskriteriums verbessert auswählbar sind.

Das KI-Modul kann eine Methode des Deep Learnings (mittels künstlicher neuronaler Netze) einsetzen, bei der mehrere Schichten künstlicher Neuronen die Eingangsgrößen (Merkmalsvektor) mit der Ausgangsgröße (Klassifikation, Regression...) verknüpfen. Ebenso können zahlreiche andere Machine Learning Methoden, Random Forest Algorithmen (randomisierte Entscheidungsbäume) oder Support Vector Machines (Schätzung mittels Stützvektoren im Vektorraum der Merkmalsvektoren) eingesetzt werden, insbesondere um den Rechenaufwand zu begrenzen. Das KI-Modul wird prinzipiell mit medizinischen Daten und/oder mit Daten aus einer Expertendatenbank trainiert.

Bevorzugterweise ist das Künstliche Intelligenzmodul ausgebildet, das Auswählen der Daten im Schritt f) zu optimieren. Dabei verarbeitet das KI-Modul die statistischen medizinischen Daten der mehreren Individuen mit einer optimierten Geschwindigkeit, sodass das Ergänzen der ausgewählten Daten beschleunigt wird.

Alternativ oder ergänzend ist das Künstliche Intelligenzmodul ausgebildet, das Festlegen der fehlenden Dateneinträge im Schritt e) zu optimieren. Dabei verarbeitet das KI-Modul die statistischen medizinischen Daten der mehreren Individuen mit einer optimierten Geschwindigkeit, sodass der Vergleich der analysierten Daten mit dem festgelegten Datenvervollständigungskriterium und das Festlegen von fehlenden Dateneinträgen für den digitalen Zwilling auf Basis des Datenvervollständigungskriteriums beschleunigt ist.

Vorzugsweise ist mindestens eine medizinische Untersuchungseinrichtung vorhanden, welche mit der Schnittstelle zum Austausch von Daten verbunden ist. Eine derartige Untersuchungseinrichtung ist beispielsweise das Pathopsy^{™} Center. Dabei handelt es sich um eine Einrichtung zum Erfassen von medizinischen oder physischen Daten von Individuen. Dieses hat zum Ziel, von jedem verstorbenen Individuum Daten zu gewinnen, zumindest von jedem Individuum, der in einem Klinikum verstarb (beispielsweise ca. 75% aller Menschen in Österreich). Damit ist ein sehr rascher Aufbau statistischer Daten möglich, die für das Vervollständigen eines digitalen Zwillings genützt werden können.

Alternativ oder ergänzend ist mindestens eine medizinische Untersuchungseinrichtung vorhanden, welche mit der Schnittstelle zum Austausch von Steuerbefehlen verbunden ist. Damit kann das System direkt auf die Steuerung der medizinischen Untersuchungseinrichtung zugreifen und insbesondere fehlende medizinische Daten von lebenden oder verstorbenen Individuen für den Datensatz des definierten Individuums beschaffen.

Bevorzugterweise ist das Datenauswertemodul ausgebildet, Steuerbefehle für zumindest eine medizinische Untersuchungseinrichtung zu erstellen. Damit kann das Datenauswertemodul direkt auf die Steuerung der medizinischen Untersuchungseinrichtung zugreifen und insbesondere fehlende medizinische Daten für das definierte Individuum beschaffen.

Ein erfindungsgemässes computerimplementiertes Verfahren zum Erstellen eines digitalen Zwillings eines Individuums umfasst mindestens die folgenden Schritte:
a) Bereitstellen eines Templates für einen digitalen Zwilling eines definierten Individuums;
b) Festlegen eines Datenvervollständigungskriteriums für den digitalen Zwilling des definierten Individuums;
c) Aufnehmen von Daten, insbesondere medizinischen Daten, des definierten Individuums in den digitalen Zwilling;
d) Analysieren der aufgenommenen Daten des definierten Individuums im digitalen Zwilling;
e) Vergleichen der analysierten Daten mit dem festgelegten Datenvervollständigungskriterium und Festlegen von fehlenden Dateneinträgen für den digitalen Zwilling auf Basis des Datenvervollständigungskriteriums;
f) Auswählen von Daten aus statistischen medizinischen Daten von mehreren Individuen aus dem zumindest einen Datenspeichermedium;
g) Ergänzen der fehlenden Dateneinträge im digitalen Zwilling des definierten Individuums mit den ausgewählten Daten.

Dieses computerimplementierte Verfahren ersetzt bzw. reduziert klinische Studien an Lebewesen massiv. Zur Schaffung des digitalen Zwillings beispielsweise eines bestimmten Menschen werden zunächst existierende Daten dieses Menschen (z.B. aus Krankenakte aber auch andere Informationen wie z.B. Tätigkeit, Wohnorte, usw.) verwendet. Um Datenlücken aufzufüllen, werden dann zusätzlich statistisch relevante medizinische Daten von mehreren möglichst ähnlichen Individuen verwendet. Damit wird eine Erprobung von neuen Therapien am digitalen Zwilling ermöglicht sowie eine parallele Analyse an vielen Instanzen desselben digitalen Zwillings geschaffen. Die statistischen medizinischen Daten stammen von vielen unterschiedlichen Individuen aus unterschiedlichen Krankenhäusern, Ländern oder Kontinenten.

Durch die Nutzung dieses Systems können beispielsweise an digitalen Zwillingen von Menschen viele Studien mit zumindest gleichwertiger Ergebnisqualität zu heutigen klinischen Studien an lebenden Menschen durchgeführt werden. Für die Studien können anstelle echter Individuen ein oder mehrere digitale Zwillinge verwendet werden. Ein digitaler Zwilling eines Menschen muss nicht zwangsläufig identisch zum Menschen sein, sondern kann auch statistischen relevante Daten anderer Menschen beinhalten. Das computerimplementierte Verfahren ist auf einem Computer, wie einer Recheneinheit oder einem Prozessor, ausführbar.

Ein erfindungsgemässes Computerprogrammprodukt umfasst Programmbefehle, die bei einer Ausführung des Computerprogramms durch einen Computer, ein hier offenbartes System veranlassen, ein hier vorliegend beschriebenes Verfahren auszuführen. Das Computerprogrammprodukt kann an einer Recheneinheit ausgeführt werden, und somit die Programmbefehle schrittweise abarbeiten, um die ausgewählten Daten an einer Schnittstelle bereitzustellen. Diese können dann einem Benutzer oder einem KI-Modul bereitgestellt werden, um insbesondere eine Untersuchungseinrichtung zu steuern.

Ein erfindungsgemässes computerlesbares Speichermedium, das zumindest ein Computerprogrammprodukt umfasst, das bei der Ausführung durch zumindest eine Recheneinheit dieses veranlasst, zumindest ein hier vorliegend beschriebenes Verfahren durchzuführen.

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung beschrieben sind.

Die Bezugszeichenliste ist wie auch der technische Inhalt der Patentansprüche und Figuren Bestandteil der Offenbarung. Die Figuren werden zusammenhängend und übergreifend beschrieben. Gleiche Bezugszeichen bedeuten gleiche Bauteile, Bezugszeichen mit unterschiedlichen Indices geben funktionsgleiche oder ähnliche Bauteile an.

Mittels der nachfolgenden Figuren wird anhand von Ausführungsbeispielen die Erfindung näher erläutert.

Positionsangaben, wie "oben", unten", "rechts" oder "links" sind jeweils auf die entsprechenden Darstellungen bezogen und sind nicht als einschränkend zu verstehen.

Obwohl die Erfindung mittels der Figuren und der zugehörigen Beschreibung dargestellt und detailliert beschrieben ist, sind diese Darstellung und diese detaillierte Beschreibung illustrativ und beispielhaft zu verstehen und nicht als die Erfindung einschränkend. Es versteht sich, dass Fachleute Änderungen und Abwandlungen machen können, ohne den Umfang der folgenden Ansprüche zu verlassen. Insbesondere umfasst die Erfindung ebenfalls Ausführungsformen mit jeglicher Kombination von Merkmalen, die vorstehend zu verschiedenen Aspekten und/oder Ausführungsformen genannt oder gezeigt sind.

Die Erfindung umfasst ebenfalls einzelne Merkmale in den Figuren, auch wenn sie dort im Zusammenhang mit anderen Merkmalen gezeigt sind und/oder vorstehend nicht genannt sind. Im Weiteren schliesst der Ausdruck "umfassen" und Ableitungen davon andere Elemente oder Schritte nicht aus. Ebenfalls schliesst der unbestimmte Artikel "ein" beziehungsweise "eine" und Ableitungen davon eine Vielzahl nicht aus. Die Funktionen mehrerer in den Ansprüchen aufgeführter Merkmale können durch eine Einheit erfüllt sein. Die Begriffe "im Wesentlichen", "etwa", "ungefähr" und dergleichen in Verbindung mit einer Eigenschaft beziehungsweise einem Wert definieren insbesondere auch genau die Eigenschaft beziehungsweise genau den Wert. Alle Bezugszeichen in den Ansprüchen sind nicht als den Umfang der Ansprüche einschränkend zu verstehen.

### Figurenbeschreibung

Die Figuren werden zusammenhängend und übergreifend beschrieben. Gleiche Bezugszeichen bedeuten gleiche Bauteile. Es zeigen
Fig. 1: ein erstes erfindungsgemässes System zum Erstellen eines digitalen Zwillings eines Individuums in einer schematischen Darstellung;
Fig. 2: ein weiteres erfindungsgemässes System zum Erstellen eines digitalen Zwillings eines Individuums in einer schematischen Darstellung; und
Fig. 3: ein erfindungsgemässes computerimplementiertes Verfahren mit einem System gemäss Fig. 1 in einem Flussdiagramm.

### Ausführung der Erfindung

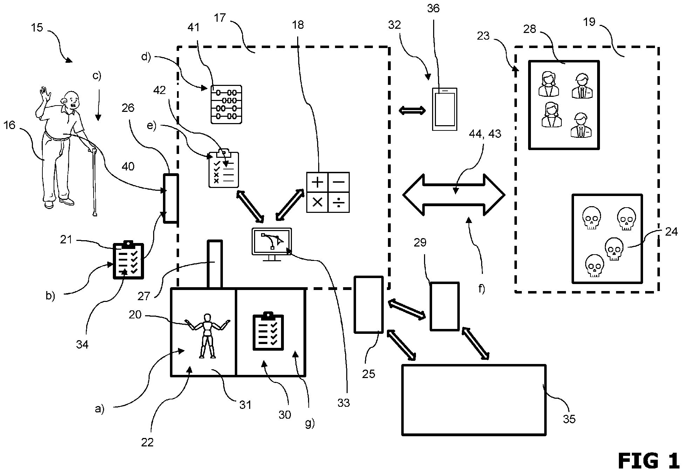

**Figur** 1 zeigt eine erste Ausführungsform eines Systems 15 zum Erstellen eines digitalen Zwillings 22 eines Individuums 16 mithilfe von Daten. Das System 15 umfasst ein Datenauswertemodul 17 mit einer Recheneinheit 18, welches zum Datenaustausch mit einem Datenspeichermedium 19 verbunden ist, wobei das Datenauswertemodul 17 dazu ausgebildet ist, beim Erstellen des digitalen Zwillings 22 mindestens die folgenden Schritte auszuführen:
a) Bereitstellen eines Templates 20 für einen digitalen Zwilling 22 eines definierten Individuums 16;
b) Festlegen eines Datenvervollständigungskriteriums 21 für den digitalen Zwilling 22 des definierten Individuums 16;
c) Aufnehmen von Daten 40 des definierten Individuums 16 in den digitalen Zwilling 22;
d) Analysieren der aufgenommenen Daten 40 des definierten Individuums 16 im digitalen Zwilling 22;
e) Vergleichen der analysierten Daten 41 mit dem festgelegten Datenvervollständigungskriterium 21 und Festlegen von fehlenden Dateneinträgen 42 für den digitalen Zwilling 22 auf Basis des Datenvervollständigungskriteriums 21;
f) Auswählen von Daten 43 aus statistischen medizinischen Daten 44 von mehreren Individuen 23 aus dem Datenspeichermedium 19;
g) Ergänzen der fehlenden Dateneinträge im digitalen Zwilling 22 des definierten Individuums 16 mit den ausgewählten Daten 43.

Zur Schaffung des digitalen Zwillings 22, hier vorliegend eines bestimmten Menschen, werden zunächst existierende Daten dieses Menschen aus der Krankenakte und auch andere Informationen wie z.B. Tätigkeit, Wohnorte, usw. verwendet und in den digitalen Zwilling 22 eingespeist. Um Datenlücken aufzufüllen, werden dann zusätzlich Daten 43 von mehreren Individuen 23 aus statistischen medizinischen Daten 44 erzeugt bzw. verwendet.

Das System 15 weist einen Auswertealgorithmus 33 auf, der die aufgenommenen Daten 40 im Schritt d) auf Basis des Datenvervollständigungskriteriums 21 analysiert. Der Auswertealgorithmus 33 kann in der Recheneinheit 18 ausgeführt werden und ist beispielsweise ausgebildet, zumindest einen Biomarker zu identifizieren. Darüber hinaus ist der Auswertealgorithmus 33 ausgebildet, den Vergleich der Daten in Schritt e) zu optimieren. Dafür weist der Auswertealgorithmus 33 Rahmenbedingungen und Schranken auf, die den Vergleich der Daten verbessert und damit optimiert. Der Auswertealgorithmus 33 kann die zur Verfügung stehenden aufgenommenen Daten 40 nach zusätzlichen Parametern, wie etwa Ort und Zeitpunkt eines Ereignisses, insbesondere der Zeitpunkt einer Krankheitsdiagnose beim Individuum 16, reihen, und damit Daten mit früheren Diagnosezeitpunkten in der Auswahl der statistischen medizinischen Daten 44 bevorzugen.

Alle relevanten ausgewählten Daten 43 werden an einer Schnittstelle 25 bereitgestellt, wobei die Schnittstelle 25 mit einem Touchscreen 31 als erste Anzeigeeinrichtung 30 verbunden ist. Das System 15 weist eine Steuerungseinrichtung 29 auf, wobei die Steuerungseinrichtung 29 die Schnittstelle 25 veranlasst, die ausgewählten Daten 43 oder ergänzend die analysierten Daten 41 auszugeben. Der Touchscreen 31 ist mit einem Graphikmodul 27 verbunden, um den digitalen Zwilling 22 graphisch darzustellen, und insbesondere animierbar darzustellen. Der Benutzer kann mithilfe des Touchscreens 31 zudem weitere Handlungen vornehmen oder Korrekturen im zumindest einen Datenvervollständigungskriterium 21 vornehmen, um zu den gewünschten statistischen medizinischen Daten 44 mit der gewünschten Relevanz zu kommen. Weitere Anzeigeeinrichtungen 32 wie beispielsweise ein Smartphone 36 oder ein Tablet sind mit dem System 15 verbunden, um die Daten 40, 41, 42, 43, 44 vielen Benutzern des Systems 15 zur Verfügung zu stellen. Vor dem Auswählen der Daten aus den statistischen medizinischen Daten 44 von mehreren Individuen 23 im Schritt f) werden diese Daten 43 im Datenauswertemodul 17 verifiziert. Die dargestellten Pfeile zeigen beispielhaft einen Austausch der zuvor genannten Daten mit der Steuerungseinrichtung 29, dem Datenspeichermedium 19 oder der Anzeigeeinrichtungen 32.

Der Benutzer des Systems 15 definiert ein oder mehrere Datenvervollständigungskriterien 21. Dabei werden Einschränkungen der allgemeinen Zieldefinition, z.B. auf bestimmte Personengruppen (Alter, Geschlecht, Anzahl, etc.) spezifiziert und über eine Eingabeeinrichtung 26 ins System 15 eingeben. Im vorliegenden Beispiel verlangt das Datenvervollständigungskriterium 21, dass vergleichbare statistische medizinische Daten 44 von einer Mindestanzahl von 100 Individuen 23 übereinstimmend vorhanden sein müssen, welche Diabetes Typ A aufweisen. Die fehlenden Dateneinträge 42 (bspw. bestimmte Blutwerte) im digitalen Zwilling 22 des definierten Individuums 16 werden nur mit den statistischen medizinischen Daten 44 der zumindest 100 Individuen ergänzt. Alternativ zu Diabetes Typ A können auch andere Krankheiten, wie beispielsweise Hepatitis, als Ausschlusskriterien bzw. Rahmenbedingen definiert werden. Das Datenvervollständigungskriterium 21 umfasst auch eine medizinische Fragestellung 34. Diese umfasst eine Texteingabe und wird datenbasiert beantwortet. Die medizinische Fragestellung 34 ist im vorliegenden Beispiel so formuliert, dass die Individuen keine Lungenvorerkrankungen, wie CoV19, aufweisen dürfen. Derartige Daten von Individuen werden vom System 15 ausgeschlossen.

Die statistischen medizinischen Daten 44 umfassen Daten von verstorbenen Individuen 24 und von lebenden Individuen 28. Unter Daten verstorbener Individuen 24 werden zum einen Autopsiedaten verstanden und zum anderen historische Patientendaten bzw. die Patientenakte eines Verstorbenen, bzw. historische Datensätze, die die Krankengeschichte eines Verstorbenen und alle damit in Verbindung stehenden Daten des verstorbenen Individuums widerspiegeln. Die lebenden Individuen 28 weisen unter anderem auch medizinische Daten auf, welche statistisch gesehen zum digitalen Zwilling 22 des definierten Individuums 16 passen. Beispielsweise enthält das Datenspeichermedium 19 auch medizinische Daten, die aufgrund des Alters, des Geschlechts, der Vorerkrankungen und des Blutbildes mit dem digitalen Zwilling des definierten Individuums übereinstimmen.

Das System 15 weist ein Künstliches I ntelligenzmodul (KI) 35 auf, welches auf Basis des Datenvervollständigungskriteriums 21 die Ergänzung des digitalen Zwillings 22 des definierten Individuums 16 mit Daten vorschlägt. Die ausgewählten statistischen medizinischen Daten 44 werden wiederum als Trainingsdaten für das KI-Modul 35 eingesetzt, sodass das System 15 intelligent und selbstlernend ist. Weiters weist das Kl-Modul 35 den statistischen medizinischen Daten 44 eine medizinische Relevanz auf Basis des Datenvervollständigungskriteriums 21 zu und verknüpft somit die statistischen medizinischen Daten 44 mit dem Datenvervollständigungskriterium 21. Das KI-Modul 35 kann wiederum ausgebildet sein, den statistischen medizinischen Daten 44 eine medizinische Relevanz zuzuordnen, sodass diese bei einer nachfolgenden veränderten Bestimmung eines Datenvervollständigungskriteriums verbessert auswählbar sind. Beispielsweise umfasst die medizinische Relevanz der statistischen medizinischen Daten 44 eine Aussage über den Grad der Verengung der Herzgefässe, wenn die Wahrscheinlichkeit eines Infarkts festgestellt werden soll.

In einer ergänzenden Ausführungsform eines Systems gemäss dem System 15 ist der Auswertealgorithmus ausgebildet, zumindest einen medizinischen Behandlungsvorschlag für das definierte Individuum 16 bereitzustellen.

In einer ergänzenden Ausführungsform eines Systems gemäss dem System 15 ist das künstliche Intelligenzmodul 35 ausgebildet, das Auswählen der Daten 43 im Schritt f) zu optimieren. Dabei verarbeitet das KI-Modul 35 die statistischen medizinischen Daten 44 von mehreren Individuen mit einer optimierten Geschwindigkeit.

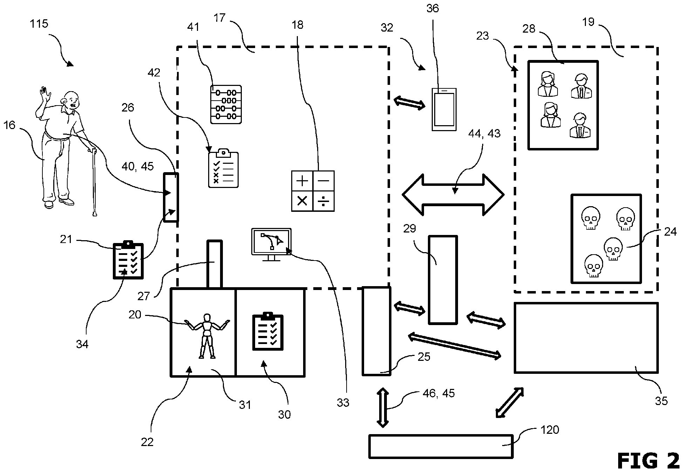

**Figur 2** zeigt eine weitere Ausführungsform eines System 115 zum Erstellen eines digitalen Zwillings 22 eines Individuums 16, wobei das System 115 gemäss seiner funktionellen und strukturellen Merkmale dem System 15 gemäss **Figur 1** entspricht und zusätzlich eine medizinische Untersuchungseinrichtung 120 vorhanden ist, welche mit der Schnittstelle 25 zum Austausch von medizinischen Daten 45 verbunden ist. Eine derartige Untersuchungseinrichtung 120 ist beispielsweise das Pathopsy^{™} Center. Dieses hat zum Ziel, von jedem verstorbenen Individuum medizinische Daten 45 zu gewinnen. Die medizinische Untersuchungseinrichtung 120 ist mit der Schnittstelle 25 zum Austausch von Steuerbefehlen 46 verbunden. Damit kann das System 115 direkt auf die Steuerung der medizinischen Untersuchungseinrichtung 120 zugreifen und insbesondere fehlende Daten bzw. Dateneinträge 42 von dem definierten Individuum 16 beschaffen. Dabei ist das Datenauswertemodul 17 ausgebildet, Steuerbefehle 46 für die medizinische Untersuchungseinrichtung 120 zu erstellen. Die dargestellten Pfeile zeigen beispielhaft einen Austausch der zuvor genannten Daten mit der Steuerungseinrichtung 29, dem Datenspeichermedium 19, dem KI-Modul 35, der medizinischen Untersuchungseinrichtung 120 oder der Anzeigeeinrichtungen 32.

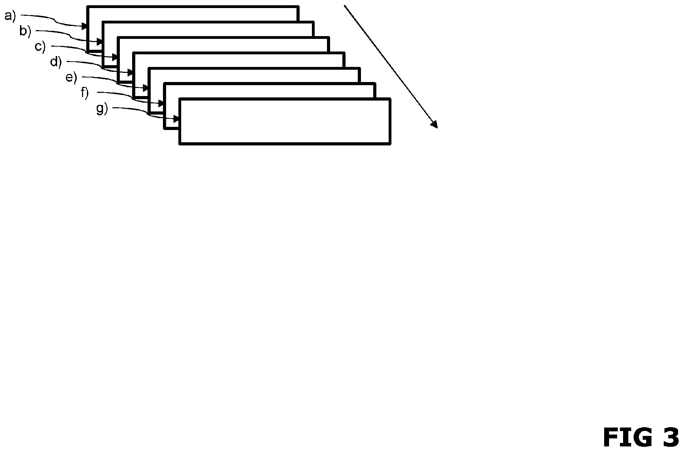

**Figur 3** zeigt eine Ausführungsform eines computerimplementierten Verfahrens zum Erstellen eines digitalen Zwillings 22 eines Individuums 16 in einem Flussdiagramm, welches in einem System 15 gemäss der **Figur 1** ausführbar ist und umfasst mindestens die folgenden Schritte:
a) Bereitstellen eines Templates für einen digitalen Zwilling eines definierten Individuums;
b) Festlegen eines Datenvervollständigungskriteriums für den digitalen Zwilling des definierten Individuums;
c) Aufnehmen von Daten, insbesondere medizinischen Daten, des definierten Individuums in den digitalen Zwilling;
d) Analysieren der aufgenommenen Daten des definierten Individuums im digitalen Zwilling;
e) Vergleichen der analysierten Daten mit dem festgelegten Datenvervollständigungskriterium und Festlegen von fehlenden Dateneinträgen für den digitalen Zwilling auf Basis des Datenvervollständigungskriteriums;
f) Auswählen von Daten aus statistischen medizinischen Daten von mehreren Individuen aus dem zumindest einen Datenspeichermedium;
g) Ergänzen der fehlenden Dateneinträge im digitalen Zwilling des definierten Individuums mit den ausgewählten Daten.

Ein Computerprogrammprodukt kann in der Recheneinheit 18 eines Computers im System 15 abgelegt sein und umfasst Programmbefehle, die bei einer Ausführung des Computerprogramms durch den Computer, den Computer dazu veranlassen, ein hier vorliegend beschriebenes Verfahren auszuführen.

### Bezugszeichenliste

- 15: System
- 16: definiertes Individuum
- 17: Datenauswertemodul
- 18: Recheneinheit
- 19: Datenspeichermedium
- 20: Template
- 21: Datenvervollständigungskriterium
- 22: digitaler Zwilling
- 23: mehrere Individuen
- 24: verstorbene Individuen
- 25: Schnittstelle
- 26: Eingabeeinrichtung
- 27: Graphikmodul
- 28: lebende Individuen
- 29: Steuerungseinrichtung
- 30: erste Anzeigeeinrichtung
- 31: Touchscreen
- 32: weitere Anzeigeeinrichtungen
- 33: Auswertealgorithmus
- 34: medizinische Fragestellung
- 35: KI-Modul
- 36: Smartphone
- 40: aufgenommene Daten
- 41: analysierte Daten
- 42: fehlende Daten/Dateneinträge
- 43: ausgewählte Daten
- 44: statistische medizinische Daten
- 45: medizinische Daten
- 46: Steuerbefehle

- 115: System
- 120: medizinische Untersuchungseinrichtung

## Patentansprüche

1. System (15; 115) zum Erstellen eines digitalen Zwillings (22) eines Individuums (16), insbesondere eines Menschen oder eines Tieres, mithilfe von Daten umfassend
ein Datenauswertemodul (17) mit zumindest einer Recheneinheit (18), welches zum Datenaustausch mit zumindest einem Datenspeichermedium (19) verbunden ist, wobei das Datenauswertemodul (17) dazu ausgebildet ist, beim Erstellen des digitalen Zwillings (22) mindestens die folgenden Schritte auszuführen, wobei insbesondere die Reihenfolge der folgenden Schritte festgelegt sind:
a) Bereitstellen eines Templates (20) für einen digitalen Zwilling (22) eines definierten Individuums (16);
b) Festlegen zumindest eines Datenvervollständigungskriteriums (21) für den digitalen Zwilling (22) des definierten Individuums (16);
c) Aufnehmen von Daten, insbesondere medizinische Daten, des definierten Individuums (16) in den digitalen Zwilling (22);
d) Analysieren der aufgenommenen Daten des definierten Individuums (16) im digitalen Zwilling (22);
e) Vergleichen der analysierten Daten mit dem festgelegten Datenvervollständigungskriterium (21) und Festlegen von fehlenden Dateneinträgen für den digitalen Zwilling (22) auf Basis des Datenvervollständigungskriteriums (21);
f) Auswählen von Daten aus statistischen medizinischen Daten von mehreren Individuen (23) aus dem zumindest einen Datenspeichermedium (19);
g) Ergänzen der fehlenden Dateneinträge im digitalen Zwilling (22) des definierten Individuums (16) mit den ausgewählten Daten.

2. System nach Anspruch 1, **dadurch gekennzeichnet, dass** das Datenvervollständigungskriterium (21) zumindest eine medizinische Fragestellung (34) umfasst.

3. System nach Anspruch 1, oder 2, **dadurch gekennzeichnet, dass** eine Schnittstelle (25) vorhanden ist, an der die Daten des digitalen Zwillings (25) für einen Benutzer bereitstellbar sind und/oder an einer Anzeigeeinrichtung (30), insbesondere animiert, darstellbar sind, wobei eine Steuerungseinrichtung (29) vorhanden ist und die Steuerungseinrichtung (29) die Schnittstelle (25) und/oder die Anzeigeeinrichtung (30) veranlasst, die ausgewählten Daten auszugeben.

4. System nach einem der vorgenannten Ansprüche, **dadurch gekennzeichnet, dass** die statistischen medizinischen Daten zumindest Daten von verstorbenen Individuen (24) und/oder von lebenden Individuen (28) umfassen.

5. System nach Anspruch 4, **dadurch gekennzeichnet, dass** die Daten von verstorbenen Individuen (24) und/oder von lebenden Individuen (28) mittels zumindest eines medizinischen Datengewinnungsverfahrens generiert wurden.

6. System nach einem der vorgenannten Ansprüche, **dadurch gekennzeichnet, dass** die Daten des definierten Individuums (16) im digitalen Zwilling (22) in einer Datenstruktur im zumindest einen Datenspeichermedium (19) abgelegt sind.

7. System nach einem der vorgenannten Ansprüche, **dadurch gekennzeichnet, dass** ein Auswertealgorithmus (33) vorhanden ist, der insbesondere ausgebildet ist, zumindest einen Biomarker zu identifizieren.

8. System nach Anspruch 7, **dadurch gekennzeichnet, dass** der Auswertealgorithmus (33) ausgebildet ist, zumindest einen medizinischen Behandlungsvorschlag für das definierte Individuum (16) bereitzustellen.

9. System nach einem der vorgenannten Ansprüche, **dadurch gekennzeichnet, dass** ein Künstliches Intelligenzmodul (35) vorhanden ist, welches auf Basis des Datenvervollständigungskriteriums (21) die Ergänzung des digitalen Zwillings (22) des definierten Individuums (16) mit Daten, insbesondere automatisch, vorschlägt.

10. System nach Anspruch 9, **dadurch gekennzeichnet, dass** das Künstliche Intelligenzmodul (35) ausgebildet ist, das Auswählen der Daten im Schritt f) zu optimieren.

11. System nach Anspruch 9 oder 10, **dadurch gekennzeichnet, dass** das Künstliche Intelligenzmodul (35) ausgebildet ist, das Festlegen der fehlenden Dateneinträge im Schritt e) zu optimieren.

12. System nach einem der vorgenannten Ansprüche, **dadurch gekennzeichnet, dass** mindestens eine medizinische Untersuchungseinrichtung (120) vorhanden ist, welche mit der Schnittstelle (25) zum Austausch von Daten und/oder von Steuerbefehlen verbunden ist.

13. Computerimplementiertes Verfahren zum Erstellen eines digitalen Zwillings (22) eines Individuums (16) umfassend mindestens die folgenden Schritte:
a) Bereitstellen eines Templates für einen digitalen Zwilling eines definierten Individuums;
b) Festlegen eines Datenvervollständigungskriteriums für den digitalen Zwilling des definierten Individuums;
c) Aufnehmen von Daten, insbesondere medizinischen Daten, des definierten Individuums in den digitalen Zwilling;
d) Analysieren der aufgenommenen Daten des definierten Individuums im digitalen Zwilling;
e) Vergleichen der analysierten Daten mit dem festgelegten Datenvervollständigungskriterium und Festlegen von fehlenden Dateneinträgen für den digitalen Zwilling auf Basis des Datenvervollständigungskriteriums;
f) Auswählen von Daten aus statistischen medizinischen Daten von mehreren Individuen aus dem zumindest einen Datenspeichermedium;
g) Ergänzen der fehlenden Dateneinträge im digitalen Zwilling des definierten Individuums mit den ausgewählten Daten.

14. Computerprogrammprodukt umfassend Programmbefehle, die bei einer Ausführung des Computerprogramms durch einen Computer, ein System gemäss einem der Ansprüche 1 bis 12 veranlassen, ein Verfahren nach Anspruch 13 auszuführen.

15. Computerlesbares Speichermedium, das zumindest ein Computerprogrammprodukt umfasst, das bei der Ausführung durch zumindest eine Recheneinheit dieses veranlasst, zumindest ein Verfahren nach Anspruch 13 durchzuführen.
